# EUROPEAN PATENT APPLICATION

(11) **EP 3 992 980 A1**
(43) Date of publication of application: **04.05.2022**
(21) Application number: 20204552.2
(22) Date of filing: 29.10.2020
(51) Int. Cl.: G16H 30/40, G16H 50/20

(54) **MEDICAL IMAGE POST-PROCESSING**

(71) Applicant: Koninklijke Philips N.V., 5656 AG Eindhoven (NL)
(72) Inventor: GOOßEN, André, 5656 AE Eindhoven (NL); NICKISCH, Hannes, 5656 AE Eindhoven (NL)
(74) Representative: Philips Intellectual Property & Standards

(57) **Abstract**

The present disclosure provides means for improved medical image post-processing. It utilizes an image data post-processing mechanism (122) comprising an encoder (122A) configured to encode input image data with input image properties and a decoder (122B) configured to decode the input image data to provide output image data with output image properties different to the input image properties. The post-processing mechanism (122) comprises a first post-processing setting applied to the encoder and/or the decoder and assigned to first output image properties. Further, the post-processing mechanism (122) is configured to predict a second post-processing setting applicable to the encoder and/or the decoder and assigned to second output image properties. The post-processing mechanism (122) is further configured to provide an image proposal comprising the input image data post-processed with the predicted second post-processing setting. Furthermore, the post-processing mechanism (122) is configured to receive a feedback signal assigned to the image proposal, and to evaluate the predicted second post-processing setting based on the received feedback signal.

## Description

### FIELD OF THE INVENTION

The present invention relates to medical imaging. In particular, the present disclosure relates to an apparatus for medical image post-processing, a system for medical image post-processing, a method for medical image post-processing, a computer program element, and a computer-readable medium.

### BACKGROUND OF THE INVENTION

Particularly in medical imaging, the best possible image quality is desirable, for example to enable good legibility of the images, good recognition of the objects or things depicted therein and/or a good or reliable and/or fast diagnosis. Besides the image acquisition itself, the images can be post-processed to meet these quality requirements. For this purpose, various post-processing techniques can be used, which can be implemented in software, for example, and which include a number of parameters that affect the post-processing itself or the resulting image quality. The software development process can include, among other things, the modification of one or more parameters of the software or post-processing and the subsequent assessment of the resulting image quality by user feedback. This may require several image review and feedback loops with e.g. clinical experts, such as radiologists, or the like. The collected feedback may include user judgements such as perceived image quality, diagnostic relevance and/or personal taste. This process is therefore process time-consuming, and also bears the risk to loose important information on the way from the radiologist via the clinical scientist over the developer into the software, caused by the fact that at least three people are involved in this review and feedback process.

### SUMMARY OF THE INVENTION

There is, therefore, a need to improve a medical imaging system in terms of its efficiency in improving image quality. The object of the present invention is solved by the subject-matter of the appended independent claims, wherein further embodiments are incorporated in the dependent claims.

According to a first aspect, there is provided an apparatus for medical image post-processing. The apparatus comprises a data processing unit. The data processing unit is configured to utilize an image data post-processing mechanism comprising an encoder configured to encode input image data with input image properties and a decoder configured to decode the input image data to provide output image data with output image properties different to the input image properties. The post-processing mechanism comprises a first post-processing setting applied to the encoder and/or the decoder and assigned to first output image properties. The post-processing mechanism is configured to predict a second post-processing setting applicable to the encoder and/or the decoder and assigned to second output image properties. The post-processing mechanism is further configured to provide an image proposal comprising the input image data post-processed with the predicted second post-processing setting. The post-processing mechanism is further configured to receive a feedback signal, which may also referred to as feedback information, assigned to the image proposal, and to evaluate the predicted second post-processing setting based on the received feedback signal.

In this way, there is provided an automatic procedure for improving an image post-processing algorithm by automatic feedback collection and integration. In other words, there is provided a mechanism using feedback, e.g. human feedback, directly to improve image post-processing, wherein in at least some embodiments reinforcement learning is used, without the need for additional human interaction. This may avoid time-consuming and error-prone manual feedback loops by gathering feedback directly from a medical staff or clinical expert respectively, such as a radiologist or the like, and incorporating it directly into the image post-processing mechanism. Further, this may avoid the loss of information compared to several feedback loops, which may be based on the use of natural language instead of the proposed computational means using computer language.

The medical imaging may utilize X-ray imaging, for medical image post-processing, magnetic resonance imaging, or the like, to acquire one or more medical images to be post-processed.

As used herein, image post-processing, particularly medical image post-processing, may be broadly understood as editing the data captured by an image acquisition device to enhance the image in terms of e.g. quality, such as brightness, contrast, color, inserting effects, or the like. Further, post-processing may also comprise segmenting the image. In other words, post-processing may be applied on the image to improve its quality, readability and/or diagnostic value. For example, post-processing may be carried out automatically by a suitable computing means, such as a data processing unit or processor, preferably running one or more post-processing algorithms, filters, artificial networks, or the like. The post-processing mechanism as described herein may apply one or more, in some embodiments even several different, post-processing techniques to achieve enhancement of medical images as described above. Further, the post-processing mechanism may comprise that represents a supervised learning model.

Further, as used herein, the encoder and/or the decoder may be broadly understood. For example, the encoder may be configured to receive and/or encode input data into intermediate data, and may comprise a feature extractor, etc. In at least some embodiments, the encoder and/or decoder may be implemented by utilizing a support-vector machine, SVM. Alternatively or additionally, the encoder may be a suitable network, e.g. an artificial neural network, such as a fully connected neural deep network, FC, a convolutional neural network, CNN, a recurrent neural network, RNN, etc., that is configured to receive input data, such as an image, and to output a feature map, a feature vector, feature tensor, etc. The feature map, feature vector, feature tensor or the like, may be configured to hold or carry the information, the features, or the like, that represent the input data. Likewise, in at least some embodiments, the decoder may be configured to receive and/or decode the output of the encoder. In a least some embodiments, the decoder may be a suitable network, e.g. an artificial neural network, such as a fully connected neural deep network, FC, a convolutional neural network, CNN, a recurrent neural network, RNN, etc., that receives the output, e.g. feature map, a feature vector, feature tensor, etc., from the encoder and provides output data. Optionally, the decoder may comprise a similar or the same network structure as the encoder, preferably in an opposite orientation. For example, the decoder may be configured to process the output data from the encoder, and to determine an e.g. closest match to the actual input or intended output, by utilizing e.g. a loss function.

In case of utilizing an artificial neural network, such a network may comprise a plurality of layers, wherein each layer comprises one or more neurons. Neurons between adjacent layers are linked in that the outputs of neurons of a first layer are the inputs of one or more neurons in an adjacent second layer. Each such link is given a "weight" with which the corresponding input goes into an "activation function" that gives the output of the neuron as a function of its inputs. The activation function is typically a nonlinear function of its inputs. For example, the activation function may comprise a "pre-activation function" that is a weighted sum or other linear function of its inputs, and a thresholding function or other nonlinear function that produces the final output of the neuron from the value of the pre-activation function.

The encoder and/or decoder may, optionally, be trained using records of training data. A record of training data may comprise training input data and, in at least some cases, corresponding training output data. The training output data of a record of training data is the result that is expected to be produced by the encoder and/or decoder when being given the training input data of the same record of training data as input. The deviation between this expected result and the actual result produced by the module is observed and rated by means of the "loss function". This loss function is used as a feedback for adjusting the parameters of the internal processing chain of the encoder and/or decoder. For example, the parameters may be adjusted with the optimization goal of minimizing the values of the loss function that result when all training input data is fed into the encoder and/or decoder, and the outcome is compared with the corresponding training output data.

As used herein, the first post-processing and/or second post-processing setting may be broadly understood. It may comprise or may also be referred to as e.g. one or more parameters, one or more weights, or the like, based on which the encoder and/or decoder processes its respective input data into its output data. Accordingly, an image that is processed by using the first post-processing setting may have first output image properties, such as a first quality, readability and/or diagnostic value, and/or a first segmentation, and/or one or more of a first brightness, a first contrast, a first color, first inserting effects, or the like. On the other hand, an image that is processed by using the second post-processing setting may have second output image properties, such as a second quality, readability and/or diagnostic value, and/or a second segmentation, and/or one or more of a second brightness, a second contrast, a second color, second inserting effects, or the like. Generally, using different post-processing settings may result in different image properties. For example, the one or more second post-processing settings may be rolled out globally, via e.g. remote servicing, wherein different second post-processing settings may also be provided to different sites in order to carry out e.g. preference studies, or locally by an application specialist, or randomly by altering parameters or network weights.

Further, as used herein, the image proposal may comprise one or more images that are generated by utilizing one or more different second post-processing settings. There may be an original image underlying the image proposal, wherein the original image may or may not have been post-processed with the first post-processing settings. In at least some embodiments, the original image may be taken from a worklist assigned to the user. The image proposal may then be provided for review by the user, e.g. a human, and collecting the feedback signal thereto. Additionally or alternatively, the review and/or feedback may be performed automatically, e.g. by a suitable image review and/or feedback computational technique, such as an algorithm.

Furthermore, as used herein, the feedback signal may comprise e.g. a judgement or rating of the judgement or rating of the proposed image in terms of perceived image quality, diagnostic relevance and/or personal taste on a finite ordinal, such as a degree or quality grade, or binary scale, such as good/bad, like/dislike, or a comparison between two proposed images. It is noted that this is not limited here.

According to an embodiment, the post-processing mechanism may further be configured to adapt the predicted second post-processing setting in response to the received feedback signal. In other words, the second post-processing setting may be adapted on-the-fly, and may immediately provide another proposal image. This may further improve efficiency of the process and/or image quality.

In an embodiment, the apparatus may be configured to provide the image proposal in an iterative manner comprising:
- a first iterative step, in which a first image proposal is provided and, in response thereto, a first feedback signal assigned to the first image proposal is received; and
- a second iterative step, in which the second post-processing setting is adjusted based on the first feedback signal, thereby forming a third post-processing setting; and
- a third iterative step, in which a second image proposal is provided, wherein the second image proposal is post-processed with the third post-processing setting, and a second feedback signal assigned to the second image proposal is received.

In other words, the second post-processing setting may be adapted on-the-fly, and may immediately provide another proposal image. This may further improve efficiency of the process and/or image quality.

According to an embodiment, the post-processing mechanism may further comprise a machine learning predictor configured to utilize a reinforcement learning technique to provide the prediction of the second post-processing setting and/or directly the post-processed image proposal, and to receive the feedback signal as a reward assigned to that prediction. The machine learning predictor may also be referred to as a reinforcement learning agent. Optionally, the apparatus and/or the post-processing mechanism may be further configured to record and/or implement the prediction, i.e. the second post-processing setting dependent on the reward. In other words, the user's feedback may be translated into a numerical reward value, e.g. discrete reward steps corresponding to the user's vote. Utilizing reinforcement learning, the apparatus and/or machine learning predictor is to try to maximize the cumulative reward and thus optimize the post-processing setting according to the user's feedback. This may further improve the efficiency of the developing process and/or the image quality.

In an embodiment, the machine learning predictor may further be configured to predict the second post-processing setting and/or directly the post-processed image proposal in a manner to maximize the reward expected. For example, the machine learning predictor may comprise and/or utilize one or more of an artificial neural network, a support vector machine, etc. that is preferably configured and/or trained to predict the second post-processing setting and/or directly the post-processed image proposal. Thereby, the machine learning predictor, which may be e.g. or may be referred to as a reinforcement learning agent, may interact with the user and/or the post-processing settings in discrete time steps. At each time, the predictor and/or agent may receive the current state and reward. The predictor and/or agent may then choose an action from a set of available actions, e.g. adjusting one or more parameters of the post-processing setting, which is subsequently used to generate the image proposal that is then sent to the user and/or recorded to be sent. Based on the user feedback, the post-processing mechanism is moved to a new state and the reward associated with the transition is determined. The goal of the machine learning predictor and/or reinforcement learning agent is to learn a policy which maximizes the expected cumulative reward. This may further improve the efficiency of the developing process and/or the image quality.

According to an embodiment, the feedback signal may comprise a judgement or rating of the image proposal by a user, and wherein the feedback signal is assigned to one or more of: an image quality of the image proposal, diagnostic relevance of the image proposal, personal taste of the user with regard to the image proposal. For example, collecting the user feedback may be implemented in an interactive manner, wherein the image proposal is provided to the user, e.g. displayed on a screen, together with a user prompt, e.g. integrated in a graphical user interface, and wherein the input data of the user prompt is then received by the apparatus and/or post-processing mechanism. This feedback collection may be mandatory, e.g. from time to time, or may be provided on a voluntary basis. This may further improve the efficiency of the developing process and/or the image quality.

In an embodiment, the apparatus may be configured to provide the image proposal along with a user interaction mechanism, and wherein the user interaction mechanism comprises:
- a first display comprising a representation of the image proposal;
- a second display comprising a representation of a comparison image; and
- a user prompt to capture feedback on the image proposal compared to the comparison image.

The comparison image may be a further, e.g. second, image proposal generated with a further, i.e. different and/or adjusted, post-processing setting, e.g. a third post-processing setting. This also allows relative feedback to be collected so that several image proposals can be evaluated against each other at the same time. The image proposal and the further image proposal or comparison image respectively may also be referred to as candidate images proposed. By way of example, both the image proposal and the comparison image may be shown to the user prior to collecting the feedback. Optionally, the user may be allowed to transition between both image proposals. This may further improve efficiency of the developing process and/or image quality.

According to an embodiment, the apparatus may be configured to provide the image proposal along with a user interaction mechanism, and wherein the user interaction mechanism comprises:
- a display comprising a representation of the image proposal, and a user prompt to capture feedback on the image proposal; and
- wherein the user prompt comprises one or more of: a discrete scoring mechanism, and a relative scoring mechanism.

In other words, only one image proposal is shown at one time. This may reduce the computational efforts compared to generating several image proposals.

In an embodiment, the post-processing mechanism may further be configured to receive the input image data from a picture archiving and communication system, PACS, preferably from a worklist included in the PACS, and to provide the image proposal to the PACS, preferably to a worklist included in the PACS. In other words, an image underlying the image proposal may be taken from a worklist assigned to the user. In this way, only images that would have to be studied anyway are used, and these images can then be used simultaneously to improve the post-processing mechanism. This may further improve efficiency of the developing process and/or image quality.

According to an embodiment, the apparatus may further be configured to collect the feedback signal in a data pool. For example, the feedback may be received by a central computer site, such as a server, computing cloud, or the like. Thereby, the collected feedback signal(s) may be configured to pool the feedback signal(s) and/or to employ the post-processing mechanism, and/or to adjust the post-processing setting.

In an embodiment, the data pool may be comprised by a central computing site arranged remotely to the apparatus. In this way, the post-processing setting may be managed and/or rolled out centrally.

According to a second aspect, a medical imaging system is provided. The system comprises:
- an image data source;
- an apparatus for medical image post-processing according to the first aspect; and
- a user terminal;
wherein the apparatus is operatively coupled to the image data source () and/or the user terminal.

For example, the image data source may be an image acquisition device, a picture archiving and communication system, PACS, or the like.

For example, the PACS may comprise and/or provide a worklist comprising one or more images to be studied by the user. An image underlying the image proposal, i.e. original image, may be taken from a worklist assigned to the user. In this way, only images that would have to be studied anyway are used, and these images can then be used simultaneously to improve the post-processing mechanism. This may further improve efficiency of the developing process and/or image quality.

According to a third aspect, there is provided a computer-implemented method for medical image post-processing, comprising:
- providing an image data post-processing mechanism comprising an encoder configured to encode input image data with input image properties and a decoder configured to decode the input image data to provide output image data with output image properties different to the input image properties;
- applying, by the post-processing mechanism, a first post-processing setting to the encoder and/or the decoder, wherein the first post-processing setting is assigned to first output image properties;
- predicting, by the post-processing mechanism, a second post-processing setting applicable to the encoder and/or the decoder and assigned to second output image properties;
- providing, by the post-processing mechanism, an image proposal comprising the input image data post-processed with the predicted second post-processing setting;
- receiving, by the post-processing mechanism, a feedback signal assigned to the image proposal; and
- evaluating, by the post-processing mechanism, the predicted second post-processing setting based on the received feedback signal.

As described above, regarding the first aspect, this provides an automatic procedure for improving an image post-processing algorithm by automatic feedback collection and integration.

According to an embodiment, the second post-processing setting may be applied to the encoder and/or the decoder to add, partly replace, or completely replace the first post-processing setting, if the received feedback signal indicates a reward equal to or greater than a threshold. This allows the post-processing setting to be gradually improved, depending on whether a reward is obtained and/or how high the reward is.

According to a fourth aspect, a computer program element for medical image post-processing is provided, which, when being executed by a data processing unit, is configured to control the apparatus according to the first aspect, or to control the system according to the second aspect, or to perform the method according to the third aspect.

According to a fifth aspect, there is provided a computer readable medium having stored the computer program element of the fourth aspect.

It is noted that the above embodiments may be combined with each other irrespective of the aspect involved. Accordingly, the method may be combined with structural features of the device and/or system of the other aspects and, likewise, the device and the system may be combined with features of each other, and may also be combined with features described above with regard to the method.

These and other aspects of the present invention will become apparent from and elucidated with reference to the embodiments described hereinafter.

### BRIEF DESCRIPTION OF THE DRAWINGS

Exemplary embodiments of the invention will be described in the following drawings.
Fig 1 shows in a schematic block diagram a medical imaging system for medical image post-processing according to an embodiment.
Fig. 2 shows in a schematic block diagram an apparatus for medical image post-processing comprising according to an embodiment.
Fig. 3 shows in a schematic block diagram a user terminal according to an embodiment.
Fig. 4 shows in a flow chart a method for medical image post-processing according to an embodiment.

### DETAILED DESCRIPTION OF EMBODIMENTS

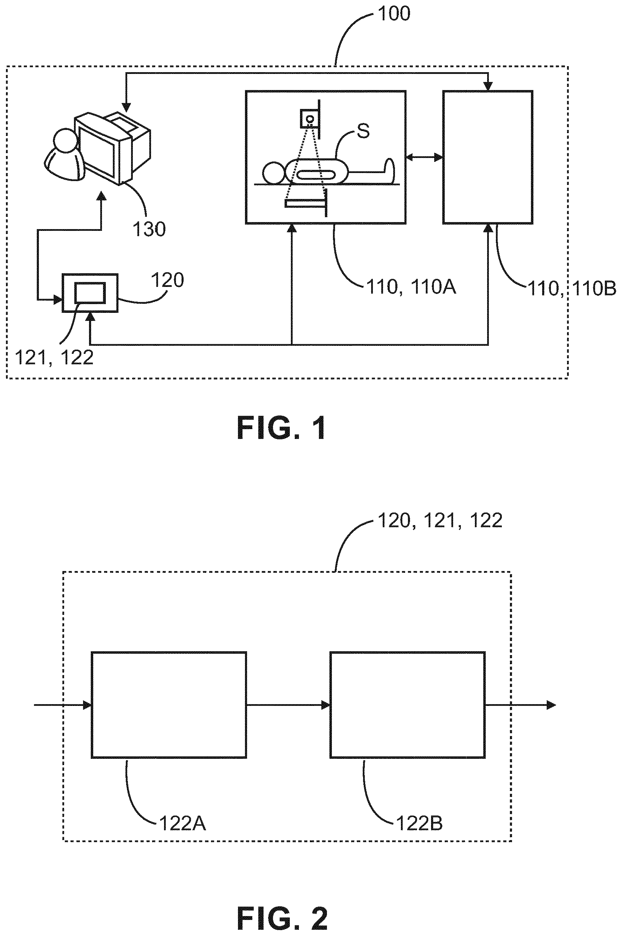

Fig. 1 illustrates in a schematic block diagram a medical imaging system 100. The medical imaging system comprises an image source 110, which, for example, may comprise a image acquisition device 110A as first image source and/or a picture archiving and communication system, PACS, 110B as a second image source, an apparatus for medical image post-processing 120, and a user terminal 130. Image acquisition devices 110A and PACS 110B are well known, so that a detailed description is omitted here. As indicated by corresponding arrows, the image source 110, 110A, 110B, the apparatus 120 and the user terminal 130 are at least partly operatively coupled, in at least communication paths as indicated by the arrows. For example, the apparatus 120 may be configured to communicate with the image source 110, 110A, 110B and the user terminal 130 to exchange data. Further, the first image source 110A may be configured to communicate with the second image source 110B, and both images sources 110A and 110B may be configured to communicate with the apparatus 120. In this way the image source 110 may provide image data to the apparatus 120. Furthermore, the user terminal 130 may be configured to communicate with the apparatus 120 and/or the second image source 110B to exchange data. Generally, the image source 110, 110A, 110B may be configured to provide one or more images to be processed, which images may also be referred to as original images.

The apparatus 120 comprises a data processing unit 121 configured to utilize, run and/or employ an image data post-processing mechanism, which, for better illustration, is designated with reference sign 122, and which may hereinafter referred to as the post-processing mechanism 122. The post processing-mechanism 122 may comprise or use one or more image post-processing techniques to generate an edited image from the original image, and may be implemented in Hardware, Software, or a combination thereof.

Now referring to Fig. 2, which shows in a schematic block diagram the apparatus 120 comprising the data processing unit 121 and the post-processing mechanism 122, the latter comprises an encoder 122A configured to encode input image data, e.g. provided by and/or received from the image source 110, 110A, 110B, with input image properties and a decoder 122B configured to decode the input image data to provide output image data with output image properties different to the input image properties. The output image data may be provided to the user terminal 130 and/or the image source 110, 110A, 110B.

The post-processing mechanism 122 comprises a first post-processing setting applied to the encoder 122A and/or the decoder 122B and assigned to first output image properties. For example, the first post-processing setting may comprise or may also be referred to as e.g. one or more parameters, one or more weights, or the like, based on which the encoder 122A and/or decoder 122B processes its respective input data into its output data.

Further, the post-processing mechanism 122 is configured to predict a second post-processing setting applicable to the encoder 122A and/or the decoder 122B and assigned to second output image properties. The first and second output image properties may differ from each other, for example, in terms of e.g. quality, such as brightness, contrast, color, inserting effects, or the like.

Furthermore, the post-processing mechanism 122 is configured to provide an image proposal comprising the input image data post-processed with the predicted second post-processing setting. Thus, if during the prediction at least one parameter has been modified or other adjustment screw, such as a weight or the like, the image proposal will differ in one or more of the image properties affected by it from a post-processed image, which, even if only hypothetically, is processed with the first post-processing setting.

Further, the post-processing mechanism 122 is configured to receive a feedback signal assigned to the image proposal form the user terminal 130, and to evaluate the predicted second post-processing setting based on the received feedback signal.

Optionally, the post-processing mechanism 122 is further configured to adapt the predicted second post-processing setting in response to the received feedback signal.

Optionally, wherein the apparatus 122 is configured to provide the image proposal in an iterative manner comprising. Thereby, in a first iterative step, a first image proposal is provided and, in response thereto, a first feedback signal assigned to the first image proposal is received. Then, in a second iterative step, the second post-processing setting is adjusted based on the first feedback signal, and, on this basis, a third post-processing setting is formed or generated. Then, in a third iterative step, a second image proposal is provided, wherein the second image proposal is post-processed with the third post-processing setting, and a second feedback signal assigned to the second image proposal is received. Depending on the feedback signal, the current post-processing setting may then either be retained or discarded, and the iteration may then either be restarted or continued until an improvement in image quality is achieved.

Optionally, the post-processing mechanism 122 further comprises a machine learning predictor configured to utilize a reinforcement learning technique to provide the prediction of the second post-processing setting and/or directly the post-processed image proposal, and to receive the feedback signal as a reward assigned to that prediction.

Optionally, the machine learning predictor is further configured to predict the second post-processing setting and/or directly the post-processed image proposal in a manner to maximize the reward expected.

Optionally, the second post-processing setting is applied to the encoder 122A and/or the decoder 122B to add, partly replace, or completely replace the first post-processing setting, if the received feedback signal indicates a reward equal to or greater than a threshold.

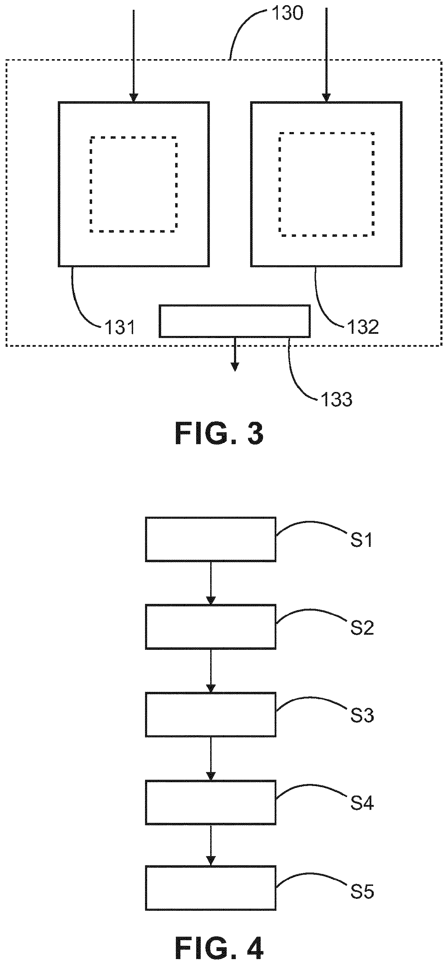

Reference is now made to Fig. 3, which shows the user terminal 130 and/or a user feedback collection and/or interaction mechanism. The user terminal 130 is configured to provide a first display 131 comprising a representation of the image proposal generated as explained above, an optional second display 132 comprising a representation of a comparison image, and a user prompt 133 to capture feedback on the image proposal compared to the comparison image. The user prompt may comprise one or more suitable input devices, such as a keyboard, mouse, or the like.

It is noted that some embodiments comprise only a single display.

Optionally, the user prompt 133 comprises one or more of a discrete scoring mechanism, and a relative scoring mechanism. Thereby, the user's feedback is translated into a numerical reward value, e.g. discrete reward steps corresponding to the user's vote.

With reference to Fig. 4, which shows a flow chart, a computer-implemented method for for medical image post-processing will be described below. The method may be implemented using the above system 100 and/or the above apparatus 120.

In a step S1, an image data post-processing mechanism, preferably the post-processing mechanism 122, applies a first post-processing setting to the encoder and/or the decoder, wherein the first post-processing setting is assigned to first output image properties.

In a step S2, the post-processing mechanism 122 predicts a second post-processing setting applicable to the encoder 122A and/or the decoder 122B and assigned to second output image properties.

In a step S3, the post-processing mechanism 122 provides an image proposal comprising the input image data post-processed with the predicted second post-processing setting;

In a step S4, the data post-processing mechanism 122 receives a feedback signal assigned to the image proposal.

In a step S5, the post-processing mechanism 122 evaluates the predicted second post-processing setting based on the received feedback signal.

Optionally, the method comprises applying the second post-processing setting to the encoder 122A and/or the decoder 122B to add, partly replace, or completely replace the first post-processing setting, if the received feedback signal indicates a reward equal to or greater than a threshold. This may be constitute or may be a part of the evaluation as described above.

In another exemplary embodiment, a computer program or computer program element is provided that is characterized by being configured to execute the method steps of the method according to one of the preceding embodiments, on an appropriate system.

The computer program element might therefore be stored on a data processing unit, which might also be part of an embodiment. This data processing unit may be configured to perform or induce performing of the steps of the method described above. Moreover, it may be configured to operate the components of the above described device and/or system. The computing unit can be configured to operate automatically and/or to execute the orders of a user. A computer program may be loaded into a working memory of a data processor. The data processor may thus be equipped to carry out the method according to one of the preceding embodiments.

Further, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It is noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and the foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: medical imaging system
- 110: image data source
- 120: apparatus
- 121: data processing unit
- 122: image data post-processing mechanism
- 122A: encoder
- 122B: decoder
- 130: user terminal
- 131: display
- 132: display
- 133: user prompt

## Claims

1. An apparatus (120) for medical image post-processing, comprising:
- a data processing unit (121);
- wherein the data processing unit is configured to utilize an image data post-processing mechanism (122) comprising an encoder (122A) configured to encode input image data with input image properties and a decoder (122B) configured to decode the input image data to provide output image data with output image properties different to the input image properties,
- wherein the post-processing mechanism (122) comprises a first post-processing setting applied to the encoder and/or the decoder and assigned to first output image properties,
- wherein the post-processing mechanism (122) is configured to predict a second post-processing setting applicable to the encoder and/or the decoder and assigned to second output image properties,
- wherein the post-processing mechanism (122) is further configured to provide an image proposal comprising the input image data post-processed with the predicted second post-processing setting, and
- wherein the post-processing mechanism (122) is further configured to receive a feedback signal assigned to the image proposal, and to evaluate the predicted second post-processing setting based on the received feedback signal.

2. The apparatus according to claim 1, wherein the post-processing mechanism (122) is further configured to adapt the predicted second post-processing setting in response to the received feedback signal.

3. The apparatus according to claim 1 or 2, wherein the apparatus (100) is configured to provide the image proposal in an iterative manner comprising:
- a first iterative step, in which a first image proposal is provided and, in response thereto, a first feedback signal assigned to the first image proposal is received; and
- a second iterative step, in which the second post-processing setting is adjusted based on the first feedback signal, thereby forming a third post-processing setting; and
- a third iterative step, in which a second image proposal is provided, wherein the second image proposal is post-processed with the third post-processing setting, and a second feedback signal assigned to the second image proposal is received.

4. The apparatus according to any one of the preceding claims, wherein the post-processing mechanism (122) further comprises a machine learning predictor configured to utilize a reinforcement learning technique to provide the prediction of the second post-processing setting and/or directly the post-processed image proposal, and to receive the feedback signal as a reward assigned to that prediction.

5. The apparatus according to claim 4, wherein the machine learning predictor is further configured to predict the second post-processing setting and/or directly the post-processed image proposal in a manner to maximize the reward expected.

6. The apparatus according to any one of the preceding claims, wherein the feedback signal comprises a judgement or rating of the image proposal by a user, and wherein the feedback signal is assigned to one or more of: an image quality of the image proposal, diagnostic relevance of the image proposal, personal taste of the user with regard to the image proposal.

7. The apparatus according to any one of the preceding claims, wherein the apparatus (120) is configured to provide the image proposal along with a user interaction mechanism, and wherein the user interaction mechanism comprises:
- a first display comprising a representation of the image proposal;
- a second display comprising a representation of a comparison image; and
- a user prompt to capture feedback on the image proposal compared to the comparison image.

8. The apparatus according to any one of claims 1 to 6, wherein the apparatus is configured to provide the image proposal along with a user interaction mechanism, and wherein the user interaction mechanism comprises:
- a display comprising a representation of the image proposal, and a user prompt to capture feedback on the image proposal; and
- wherein the user prompt comprises one or more of: a discrete scoring mechanism, and a relative scoring mechanism.

9. The apparatus according to any one of the preceding claims, wherein the image data post-processing mechanism (122) is further configured to receive the input image data from a picture archiving and communication system, PACS (110B), preferably from a worklist included in the PACS, and to provide the image proposal to the PACS, preferably to a worklist included in the PACS.

10. The apparatus according to any one of the preceding claims, wherein the apparatus (120) is further configured to collect the feedback signal in a data pool.

11. The apparatus according to claim 10, wherein the data pool is comprised by a central computing site arranged remotely to the apparatus (100).

12. A medical imaging system, comprising:
- an image data source (110, 110A, 110B);
- an apparatus (120) for medical image post-processing according to any one of the preceding claims; and
- a user terminal (130);
wherein the apparatus is operatively coupled to the image data source () and/or the user terminal.

13. A computer-implemented method for medical image post-processing, comprising:
- applying, by a post-processing mechanism (122), a first post-processing setting to the encoder and/or the decoder, wherein the first post-processing setting is assigned to first output image properties;
- predicting, by the post-processing mechanism (122), a second post-processing setting applicable to an encoder and/or a decoder and assigned to second output image properties;
- providing, by the post-processing mechanism (122), an image proposal comprising the input image data post-processed with the predicted second post-processing setting;
- receiving, by the post-processing mechanism (122), a feedback signal assigned to the image proposal; and
- evaluating, by the post-processing mechanism (122), the predicted second post-processing setting based on the received feedback signal.

14. The method according to claim 13, wherein the second post-processing setting is applied to the encoder and/or the decoder to add, partly replace, or completely replace the first post-processing setting, if the received feedback signal indicates a reward equal to or greater than a threshold.

15. A computer program element for medical image post-processing, which, when being executed by a data processing unit, is configured to control the apparatus according to any one of claims 1 to 11, or to control the system according to claim 12, or to perform the method according to claim 13 or 14.
